# EUROPÄISCHE PATENTSCHRIFT

(11) **EP 2 308 558 B1**
(45) Veröffentlichungstag und Bekanntmachung des Hinweises auf die Patenterteilung: **31.10.2012**
(21) Anmeldenummer: 10176655.8
(22) Anmeldetag: 14.09.2010
(51) Int. Cl.: A61N 1/365, A61N 1/37

(54) **Biventrikulärer Herzstimulator**
Biventricular cardiac stimulator
Stimulateur cardiaque bi-ventriculaire

(30) Priorität: 07.10.2009 US 249260 P
(43) Veröffentlichungstag der Anmeldung: 13.04.2011
(73) Patentinhaber: Biotronik CRM Patent AG, 6341 Baar (CH)
(72) Erfinder: Dörr, Thomas, 12437, Berlin (DE); Radtke, Torsten, 12589, Berlin (DE)
(74) Vertreter: Lindner-Vogt, Karin L.

(56) Entgegenhaltungen:
- WO-A2-02/18010
- US-A1- 2004 215 256

## Beschreibung

Die Erfindung betrifft einen biventrikulären Herzstimulator wie in Anspruch 1 definiert, mit einer rechtsventrikulären Sensingeinheit und einer linksventrikulären Sensingeinheit und einem Schrittmacherzeitgeber, der u. a. Zeitpunkte für die Abgabe von Stimulationsimpulsen, beispielsweise rechtsventrikulären oder linksventrikulären Stimulationsimpulsen, bestimmt. Die rechtsventrikuläre Sensingeinheit ist mit einer rechtsventrikulären Wahrnehmungselektrode verbunden oder kann mit einer solchen verbunden werden, während die linksventrikuläre Sensingeinheit entsprechend mit einer linksventrikulären Wahrnehmungselektrode verbunden ist oder mit einer solchen verbunden werden kann.

Biventrikuläre Herzschrittmacher sind typischerweise zur Stimulation des rechten und des linken Ventrikels eines Herzens ausgebildet, um beispielsweise eine kardiale Resynchronisationstherapie (CRT) durchzuführen. Dazu weist ein biventrikulärer Herzstimulator typischerweise eine rechtsventrikuläre Stimulationseinheit und eine linksventrikuläre Stimulationseinheit auf, die jeweils mit wenigstens einer rechtsventrikulären bzw. einer linksventrikulären Stimulationselektrode verbunden oder zu verbinden sind.

Die rechtsventrikuläre(n) Wahrnehmungselektrode(n) und die rechtsventrikuläre(n) Stimulationselektrode(n) sind dabei typischerweise an einer rechtsventrikulären Elektrodenleitung angebracht, während die linksventrikuläre(n) Wahrnehmungselektrode(n) und die linksventrikuläre(n) Stimulationselektrode(n) Bestandteile einer linksventrikulären Elektrodenleitung sind. Derartige linksventrikuläre Elektrodenleitungen sind typischerweise dazu vorgesehen, über den Coronar Sinus bis in die Nähe des linken Ventrikels vorgeschoben zu werden und werden daher auch als CS-Elektrodenleitungen bezeichnet. Bei linksventrikulären Elektrodenleitungen besteht - im Vergleich zu rechtsventrikulären Elektrodenleitungen - eine vergrößerte Wahrscheinlichkeit, dass sich die Elektrodenleitung verschiebt, so dass sich die Wahrnehmungs- bzw. Stimulationsbedingungen ändern.

Ein Schrittmacherzeitgeber bestimmt Zeitpunkte, zu denen Stimulationsimpulse an eine jeweilige Herzkammer abzugeben sind, auf Basis stimulierter oder wahrgenommener Ereignisse. Ein stimuliertes Ereignis ist die Abgabe eines Stimulationsimpulses, der zu einer Kontraktion der entsprechenden Herzkammer führt. Ein wahrgenommenes Ereignis, auch als natürliches oder intrinsisches Ereignis bezeichnet, ist eine eigenständige Kontraktion der entsprechenden Herzkammer, die über eine entsprechende Wahrnehmungselektrode erfasst wird. Die entsprechende Wahrnehmungselektrode erfasst die mit einer natürlichen Kontraktion einer entsprechenden Herzkammer einhergehenden elektrischen Potenziale. Diese werden verstärkt und durch die Schrittmachersteuerung und insbesondere den Schrittmacherzeitgeber ausgewertet.

In einem Elektrokardiogramm ist eine natürliche Kontraktion des rechten Atriums als sogenannte p-Welle zu erkennen. Das entsprechende intrinsische rechtsatriale Ereignis wird hier mit As (Atrium Sense) bezeichnet. Die natürliche Kontraktion des rechten Ventrikels äußert sich in einem Elektrokardiogramm in Form einer R-Zacke. Eine erfasste natürliche Kontraktion des rechten Ventrikels wird hier mit RVs bezeichnet. Eine erfasste natürliche Kontraktion des linken Ventrikels wird hier mit LVs bezeichnet. RVp (RV paced) bezeichnet ein stimuliertes Ereignis im rechten Ventrikel. Entsprechend bezeichnet LVp ein stimuliertes Ereignis im linken Ventrikel. Es ist bekannt, dass Herzstimulatoren für ein jeweiliges erfasstes (natürliches) Ereignis oder ein stimuliertes Ereignis jeweils ein Markersignal generieren, welches den Zeitpunkt eines jeweiligen Ereignisses charakterisiert. Der Schrittmacherzeitgeber kann dann auf diese Markersignale zurückgreifen. Markersignale werden dazu häufig in einem jeweiligen Kanal, beispielsweise einem rechtsatrialen Erfassungskanal, einem rechtsventrikulären Erfassungskanal oder einem linksventrikulären Erfassungskanal, übermittelt.

In diesem Zusammenhang ist es bekannt, Zeitpunkte für die Abgabe eines jeweiligen Stimulationsimpulses zwar zu bestimmen, die Abgabe eines Stimulationsimpulses jedoch zu unterdrücken (zu inhibieren), falls innerhalb eines bestimmten Intervalls vor dem vorgesehenen Stimulationszeitpunkt eine eigenständige Kontraktion der jeweiligen Herzkammer, also ein intrinsisches Ereignis dieser Herzkammer, erfasst wird. Wenn der Herzstimulator derart ausgebildet ist, stimuliert er die jeweilige Herzkammer nur bei Bedarf, und daher wird der entsprechende Betriebsmodus auch als Demand-Modus bezeichnet.

Wie bereits erwähnt werden die Zeitpunkte, zu denen ein jeweils nächster Stimulationsimpuls einer jeweiligen Kammer vorgesehen ist, von dem Schrittmacherzeitgeber auf Basis wahrgenommener oder stimulierter Ereignisse bestimmt. In bekannter Manier ist der Schrittmacherzeitgeber hierzu so ausgeführt, dass die Kammern eines Herzens möglichst in einer zeitlichen Reihenfolge kontrahieren, wie es auch ein gesundes Herz selbsttätig in Abhängigkeit des jeweiligen hämodynamischen Bedarfs tut. Beispielsweise folgt auf eine Kontraktion des rechten Atriums nach einer atrioventrikulären Überleitungszeit eine Kontraktion des rechten Ventrikels. Der Schrittmacherzeitgeber bestimmt in analoger Weise den Zeitpunkt für die Abgabe eines nächsten rechtsventrikulären Stimulationsimpulses anhand einer atrioventrikulären Verzögerungszeit (AVD), die mit einem stimulierten oder erfassten Ereignis im rechten Atrium ausgelöst wird. Vorteilhafterweise ist die atrioventrikuläre Verzögerungszeit derart variabel, dass sich der Schrittmacherzeitgeber möglichst optimal auf einen jeweiligen hämodynamischen Bedarf sowie an die individuellen Bedürfnisse eines jeweiligen Patienten anpassen kann.

Bei einem Herzschrittmacher, der sowohl das rechte Atrium als auch den rechten Ventrikel stimuliert, bestimmt der Schrittmacherzeitgeber auch den Zeitpunkt einer nächsten atrialen Stimulation nach einer VA-Verzögerungszeit, die sich an ein jeweiliges stimuliertes oder erfasstes ventrikuläres Ereignis anschließt und maßgeblich von einer möglichst dem hämodynamischen Bedarf angepassten Herzrate abhängt. Ein Herzstimulator, der einen Schrittmacherzeitgeber besitzt, der die Stimulationsrate an den hämodynamischen Bedarf anpassen kann, wird als ratenadaptiver Herzstimulator bezeichnet.

Bei biventrikulären Herzstimulatoren kann zusätzlich auch der linke Ventrikel stimuliert werden, um die Aktionen (Kontraktionen) des rechten Ventrikels und des linken Ventrikels im Rahmen einer kardialen Resynchronisationstherapie (CRT) zu synchronisieren. In diesem Zusammenhang spielt für den Schrittmacherzeitgeber auch eine interventrikuläre Verzögerungszeit (VVD) eine Rolle, die die zeitliche Verzögerung zwischen der vorgesehenen Abgabe eines rechtsventrikulären Stimulationsimpulses und der vorgesehenen Abgabe eines linksventrikulären Stimulationsimpulses beschreibt und auch Null oder negativ sein kann, so dass beispielsweise auch die Abgabe eines linksventrikulären Stimulationsimpulses vor der Abgabe eines rechtsventrikulären Stimulationsimpulses vorgesehen sein kann. Auch diese interventrikuläre Verzögerungszeit ist vorzugsweise in dem Sinne variabel, dass sich der Schrittmacherzeitgeber auf individuelle Bedürfnisse und momentane Anforderungen bei einem jeweiligen Patienten einstellen kann.

Da die grundsätzliche Funktionsweise biventrikulärer Herzstimulatoren einschließlich derjenigen Herzstimulatoren, die auch das rechte Atrium stimulieren können und daher Drei-Kammer-Stimulatoren sind, als bekannt vorausgesetzt werden kann, kann hier auf eine weitergehende detaillierte Beschreibung verzichtet werden.

Ein Gerät nach der Präambel von Anspruch 1 ist aus US2004 215256 bekannt.

Der Erfindung liegt die Aufgabe zugrunde, einen verbesserten Herzstimulator für die kardiale Resynchronisationstherapie zu schaffen.

Erfindungsgemäß wird diese Aufgabe durch einen biventrikulären Herzstimulator mit einer rechtsventrikulären Sensingeinheit, die einen Anschluss für eine rechtsventrikuläre Wahrnehmungselektrode aufweist oder mir einer solchen verbunden ist, einer linksventrikulären Sensingeinheit, die einen Anschluss für eine linksventrikuläre Wahrnehmungselektrode aufweist oder mir einer solchen verbunden ist, und einem Schrittmacherzeitgeber, der mit der rechtsventrikulären Sensingeinheit und der linksventrikulären Sensingeinheit verbunden ist, gelöst, wobei der Herzstimulator einen programmierbaren automatischem Umschalter aufweist, der mit dem Schrittmacherzeitgeber wirkverbunden und ausgebildet ist, den Schrittmacherzeitgeber wahlweise zwischen einer primär rechtsventrikulären Steuerung und einer primär linksventrikulären Steuerung umzuschalten, sowie einer Erfassungseinheit, die mit dem Umschalter wirkverbunden und ausgebildet ist, wenigstens einen für eine Stabilität der Elektrodenlage der linksventrikulären Wahrnehmungselektrode charakteristischen Stabilitätsparameter zu erfassen, wobei der programmierbare automatische Umschalter ausgebildet ist, die Schrittmacherzeitgebersteuerung in Abhängigkeit eines Wertes des erfassten Stabilitätsparameters automatisch umzuschalten.

Kern der Erfindung ist die automatische Umschaltung auf eine primär linksventrikuläre Steuerung. Primär linksventrikuläre Steuerung bedeutet, dass das für das Schrittmachertiming maßgebliche Ereignis von der linksventrikulären Wahrnehmungselektrode abgeleitet wird. So wird z.B. in R-synchronen Modi das Stimulationsintervall mit einem linksventrikulären (LV) Ereignis gestartet. In P-synchronen Modi wird die VA-Zeit jeweils mit einem linksventrikulären Ereignis gestartet.

Das beschriebene Verfahren zur Umschaltung von primär rechtsventrikulärer auf primär linksventrikuläre Steuerung kann auch für die entgegengesetzte Richtung eingesetzt werden, z.B. wenn die linksventrikuläre Wahrnehmungselektrode aufgrund Elektrodenbruchs oder Dislozierung keine sinnvollen oder stabilen Signale mehr liefert.

Der Erfindung liegt die Erkenntnis zugrunde, dass das Timing derzeitiger biventrikulärer Stimulatoren (CRT-Systeme) primär über die rechtsventrikuläre Wahrnehmungselektrode und, falls vorhanden, die rechtsatriale Wahrnehmungselektrode gesteuert ist. Die Signale der linksventrikulären Wahrnehmungselektrode werden nur zur Inhibierung einer linksventrikulären Stimulation im Falle einer dort wahrgenommenen Eigenaktion des linken Ventrikels verwendet. Grund für die zurückhaltende Verwendung des linksventrikulären Signals ist die höhere Dislokationsrate der linksventrikulären Elektrodenleitung - insbesondere in den ersten Wochen nach der Implantation.

Mit der zunehmenden Verbreitung von CRT-Stimulatoren wächst jedoch die Erkenntnis, dass die Signale der linksventrikulären Wahrnehmungselektrode für die Steuerung des Schrittmacherzeitgebers zu bevorzugen sind. Dies ist immer dann möglich, wenn die Position der LV-Elektrodenleitung als stabil bewertet wird (z.B. mehrere Wochen nach Implantation).

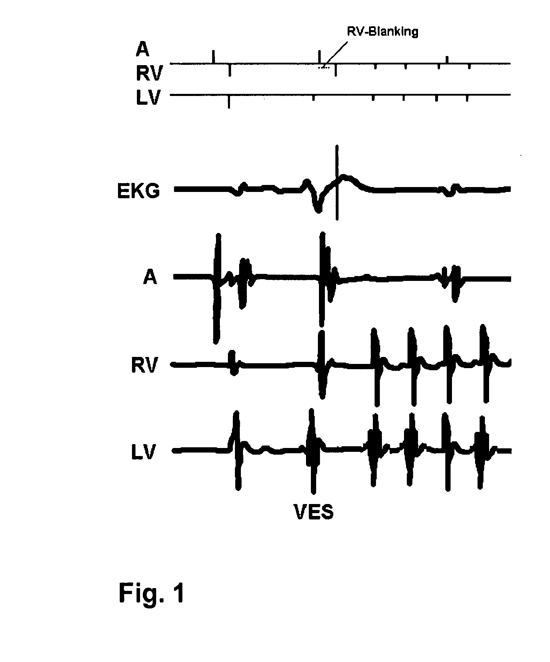

Das in Abbildung 1 gezeigte Beispiel demonstriert einen Nachteil einer rechtsventrikulär gesteuerten CRT-Stimulation. Abgebildet sind der atriale (A), rechtsventrikuläre (RV) und linksventrikuläre (LV) Markerkanal. Darunter sind das Oberflächen-EKG (EKG) und die entsprechenden intrakardialen Elektrogramme dargestellt. Aufgrund einer linksventrikulären Extrasystole (VES) wird der linke Ventrikel vorzeitig kontrahiert. Diese Erregung wird dann in den rechten Ventrikel übergeleitet. Gleichzeitig läuft der atriale Zeitgeber ab und initiiert eine atriale Stimulation. Diese Stimulation im atrialen Kanal startet immer ein Blankingintervall in den benachbarten Sensing-Kanälen (RV und LV), während dessen in diesen Kanälen keine Ereignisse erfasst werden. Aufgrund dieses Blankings kann die vom linken Ventrikel auf den rechten Ventrikel übergeleitete VES nicht wahrgenommen werden. Da die rechtsventrikuläre Erregung nicht wahrgenommen wird, wird nach Ablauf der programmierten AV-Überleitungszeit eine rechtsventrikuläre Stimulation abgegeben. Diese Stimulation erfolgt in die aufsteigende T-Welle (siehe EKG) und induziert eine ventrikuläre Tachykardie.

Vorzugsweise ist der Umschalter Bestandteil des Schrittmacherzeitgebers und so angeordnet und ausgebildet, dass der Schrittmacherzeitgeber, unabhängig vom Schaltzustand des Umschalters, sowohl auf von der rechtsventrikulären Elektrodenleitung stammende Signale als auch von der linksventrikulären Elektrodenleitung stammende Signale Zugriff hat, so dass der Schrittmacherzeitgeber die Steuerung der Stimulationstherapie von beiden Signalen abhängig machen kann. Der Umschalter bewirkt dann, dass der Schrittmacherzeitgeber primär auf die Signale einer der beiden Elektroden abstellt, so wie dies eingangs beschrieben ist. Gleichzeitig kann der Schrittmacherzeitgeber neben Ereignissen des primären Steuerkanals auch Ereignisse des jeweils anderen Kanals, z.B. zum Inhibieren von Stimulationsimpulsen, berücksichtigen.

In einer weiteren bevorzugten Ausführungsvariante, bei der die linksventrikuläre Sensingeinheit einen linksventrikulären EKG-Signalverstärker aufweist, der ein linksventrikuläres Eingangssignal für die Bewertungseinheit liefert, ist die Bewertungseinheit vorzugsweise ausgebildet, eine Maximalamplitude des linksventrikulären Eingangssignals als Stabilitätsparameter zu erfassen.

In einer ersten Untervariante ist die Bewertungseinheit weiter dazu ausgebildet, den Wert der Maximalamplitude des linksventrikulären Eingangssignals mit einem Schwellwert zu vergleichen und ein Umschalten des Umschalters von primär rechtsventrikulärer Steuerung auf primär linksventrikuläre Steuerung (und vorzugsweise auch umgekehrt) zu bewirken, wenn das linksventrikuläre Eingangssignal den vorgegebenen Schwellwert wenigstens einmal überschreitet (oder umgekehrt unterschreitet).

In diesem Zusammenhang ist es bevorzugt, wenn die Bewertungseinheit ausgebildet ist, ein Umschalten des Umschalters erst dann zu bewirken, wenn das linksventrikuläre Eingangssignal den vorgegebenen Schwellwert einer vorgegebenen Anzahl X-Mal innerhalb einer vorgegebenen Anzahl von Y Herzzyklen überschreitet. Auf diese Weise kann ausgeschlossen werden, dass ein zufälliges Überschreiten des Schwellwertes bereits eine Umschaltung von primär rechtsventrikulärer auf primär linksventrikuläre Steuerung erfolgt.

Weiterhin ist es bevorzugt, wenn die Bewertungseinheit außerdem mit einer rechtsventrikulären Sensingeinheit des biventrikulären Herzstimulators verbunden ist, die einen rechtsventrikulären EKG-Signalverstärker aufweist, der ein rechtsventrikuläres Eingangssignal für die Bewertungseinheit liefert. In diesem Fall ist die Bewertungseinheit zusätzlich dazu ausgebildet, auch eine maximale Amplitude des rechtsventrikulären Eingangssignals als weiteren Steuerparameter zu erfassen.

Bevorzugt ist hierbei eine Ausführungsvariante, bei der die Bewertungseinheit die Maximalamplitude des jeweiligen linksventrikulären Eingangssignals mit dem Wert der Maximalamplitude eines jeweiligen rechtsventrikulären Eingangssignals vergleicht und ein Umschalten des Umschalters hin zu einer primär linksventrikulären Steuerung dann bewirkt, wenn der Wert der Maximalamplitude des jeweiligen linksventrikulären Ereignisses größer ist als der Wert der Maximalamplitude des jeweiligen rechtsventrikulären Ereignisses. Die Bewertungseinheit stellt somit einen Amplitudenvergleich an und wählt dasjenige Eingangssignal für die weitere Steuerung aus, das die größere Maximalamplitude aufweist.

Zusätzlich kann die Bewertungseinheit ausgebildet sein, wenigstens ein morphologisches Signalcharakteristikum als zusätzlichen Steuerparameter auszuwerten. Geeignete morphologische Signalcharakteristika sind beispielsweise die Breite eines QX-Komplexes im linksventrikulären Eingangssignal, die Slew-Rate des linksventrikulären Eingangssignals, das Integral eines QX-Komplexes im linksventrikulären Eingangssignal und Frequenzinhalte des linksventrikulären Eingangssignals.

Bei dem Herzstimulator, der zusätzlich eine rechtsatriale Sensingeinheit aufweist, ist die Bewertungseinheit vorzugsweise auch mit dieser verbunden und dazu ausgebildet, als weiteren Stabilitätsparameter eine zeitliche Beziehung zwischen einander zugeordneten rechtsatrialen Ereignissen und linksventrikulären Ereignissen auszuwerten. Dies kann beispielsweise mit Hilfe eines Histogramms einer Vielzahl von Zeitintervallen zwischen intrinsischem atrialen Ereignis (As) und zugehörigem intrinsischen linksventrikulären Ereignis (LVs) geschehen. Ein Umschalten auf primär linksventrikuläre Steuerung kann beispielsweise dann erfolgen, wenn mehr als 95% der im Histogramm erfassten Zeitintervalle innerhalb einer definierten Zeitspanne liegen.

In einer besonders bevorzugten Ausführungsvariante ist die Bewertungseinheit dazu ausgebildet, ein Umschalten der Schrittmacherzeitgebersteuerung von 3 Kriterien abhängig zu machen, nämlich der Maximalamplitude des linksventrikulären Eingangssignals, wenigstens einem morphologischen Signalcharakteristikum und der zeitlichen Beziehung zwischen einander zugeordneten rechtsatrialen und linksventrikulären Ereignissen. Dabei kann die Bewertungseinheit dazu ausgebildet sein, ein Umschalten auf primär linksventrikuläre Steuerung bereits dann zu bewirken, wenn wenigstens zwei dieser drei Kriterien wenigstens eine vorgegebene Bedingung erfüllen. Alternativ kann die Bewertungseinheit auch ausgebildet sein, ein Umschalten erst dann zu bewirken, wenn alle drei dieser Kriterien ebenfalls wenigstens eine vorgegebene Bedingung erfüllen.

Der Schrittmacherzeitgeber ist vorzugsweise dazu ausgebildet, eine Parametertransformation durchzuführen, wenn eine Umschaltung von primär rechtsventrikulärer Steuerung auf eine primär linksventrikuläre Steuerung erfolgt oder umgekehrt, um mit der Parametertransformation sicherzustellen, dass die zeitliche Beziehung zwischen den für eine biventrikuläre Stimulation relevanten Ereignisse erhalten bleibt.

Weitere vorteilhafte Ausgestaltungen der Erfindung ergeben sich aus der nachfolgenden Beschreibung bevorzugter Ausführungsbeispiele.

Die Erfindung soll nun anhand eines Ausführungsbeispiels mit Bezug auf die Figuren näher erläutert werden. Von diesen zeigt
- Fig.1:: eine mögliche nachteilige Wirkung einer primär rechtsventrikulären Zeitsteuerung anhand eines Beispiels;
- Fig. 2:: einen 3-Kammer-Herzstimulator samt angeschlossenen Elektrodenleitungen;
- Fig. 3a:: ein schematisches Blockschaltbild einiger ausgewählter Komponenten des Herzstimulators aus Figur 2 in einer ersten Variante;
- Fig. 3b:: ein schematisches Blockschaltbild einiger ausgewählter Komponenten des Herzstimulators aus Figur 2 in einer zweiten Variante;
- Fig. 3c:: ein schematisches Blockschaltbild einiger ausgewählter Komponenten des Herzstimulators aus Figur 2 in einer dritten Variante;
- Fig. 4:: ein Beispiel einer Parametertransformation bei Umschaltung von einem primär rechtsventrikulär gesteuerten Zeitgeber zu einem linksventrikulär gesteuerten Zeitgeber; und
- Fig. 5:: den Ablauf einer automatischen Umschaltung von primär rechtsventrikulär nach primär linksventrikulär gesteuerter Stimulation nach Implantation anhand eines Beispiels.

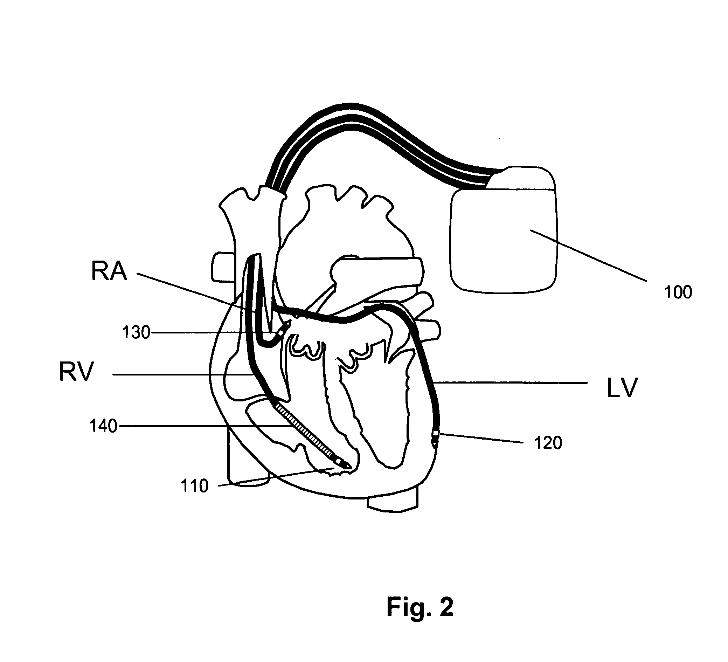

In Figur 2 ist ein 3-Kammerstimulator (100) dargestellt. Dieser ist über eine rechtsventrikuläre Elektrodenleitung mit einer Elektrode zur rechtsventrikulären Wahrnehmung und Stimulation (110) verbunden, über eine linksventrikuläre Elektrodenleitung mit einer Elektrode zur linksventrikulären Wahrnehmung und Stimulation (120), optional über eine rechtsatriale Elektrodenleitung mit einer Elektrode zur rechtsatrialen Wahrnehmung und Stimulation (130). Die rechtsventrikuläre Elektrodenleitung kann optional (falls der Herz-Herzstimulator als implantierbarer Cardioverter/Defibrillator (ICD) ausgebildet ist) mit einer Schockelektrode (140) zur Abgabe eines Defibrillationsschocks versehen sein.

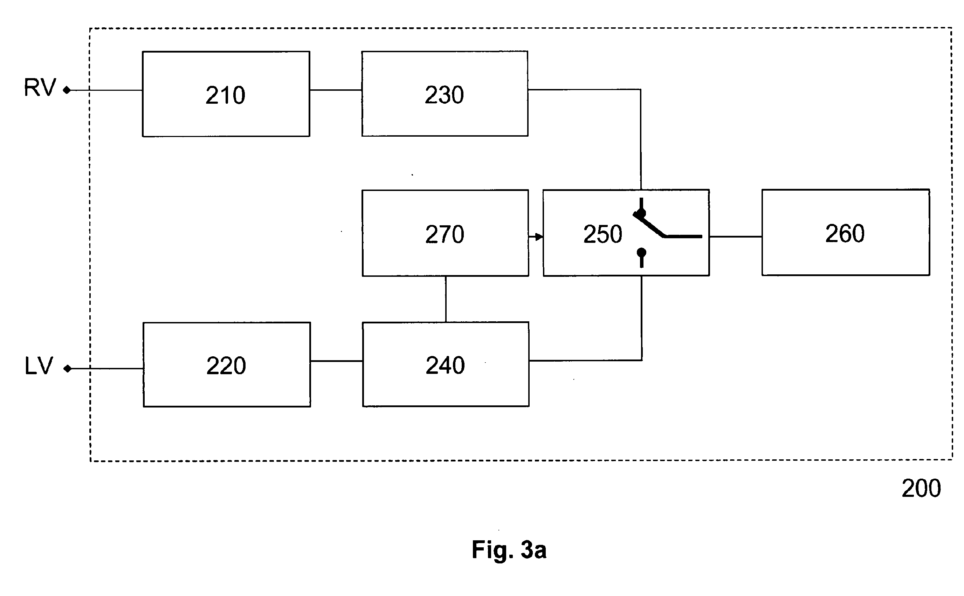

In Figur 3a ist ein Auszug aus einem Blockschaltbild eines biventrikulären Herzstimulators (200) dargestellt. Dieser Auszug zeigt nur die im Rahmen der hier beschriebenen Erfindung relevanten Komponenten des Ausführungsbeispiels. Die rechtsventrikuläre Elektrodenleitung RV und die linksventrikuläre Elektrodenleitung (LV) sind jeweils mit einer Sensingeinheit verbunden, die jeweils einen EKG-Signalverstärker (210, 220) und an diesen angeschlossen jeweils eine Sensingstufe enthalten. In den Sensingstufen (230, 240) wird der Zeitpunkt der Ventrikelerregung bestimmt, zum Beispiel durch einen Schwellwertvergleich.

Die Sensingstufen 230, 240 sind über einen programmierbaren Umschalter 250 mit einem Schrittmacherzeitgeber 260 verbunden. Mit diesem Umschalter 250 wird festgelegt, mit welchem Ereignis (RV oder LV) der Schrittmacherzeitgeber 260 gesteuert wird. Gesteuert wird dieser Umschalter 250 von einer Bewertungseinheit 270 zur Bewertung der Stabilität der Elektrodenlage der linksventrikulären Elektrodenleitung LV. Dazu ist der Umschalter 270 wenigstens mit dem EKG-Signalverstärker 220, der mit der linksventrikulären Elektrodenleitung LV verbunden ist, verbunden und erhält das Ausgangssignal des EKG-Signalverstärkers 220 als linksventrikuläres Eingangssignal.

An den Schrittmacherzeitgeber sind in üblicher Manier hier nicht dargestellte Stimulationseinheiten angeschlossen, die auf ein Signal des Schrittmacherzeitgebers hin einen Stimulationsimpuls für eine jeweilige Herzkammer erzeugen und über die entsprechende Elektrodenleitung abgeben können.

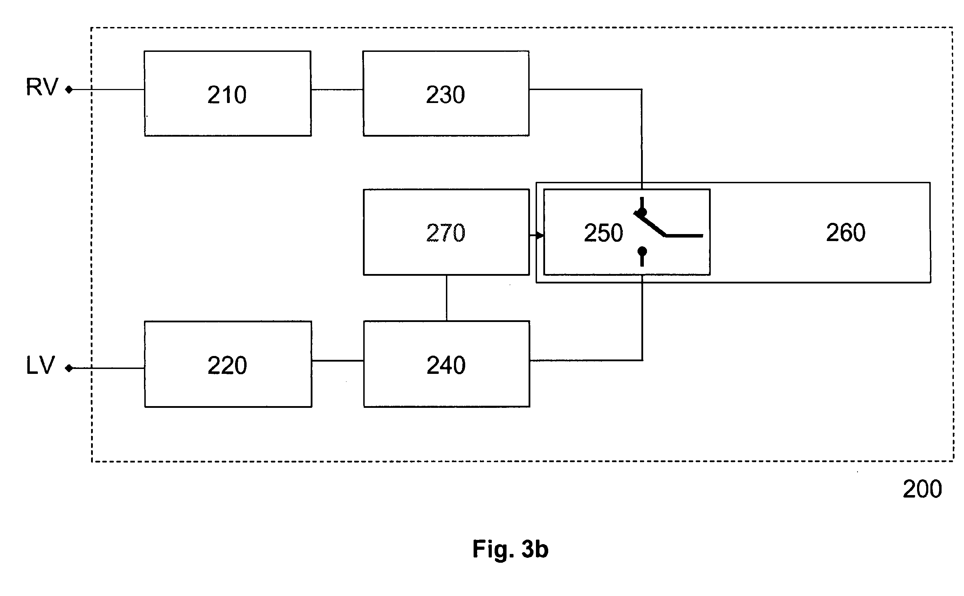

Der Umschalter 250' kann gemäß einer bevorzugten Ausführungsvariante (siehe Figur 3b) auch in den Schrittmacherzeitgeber 260' integriert sein.

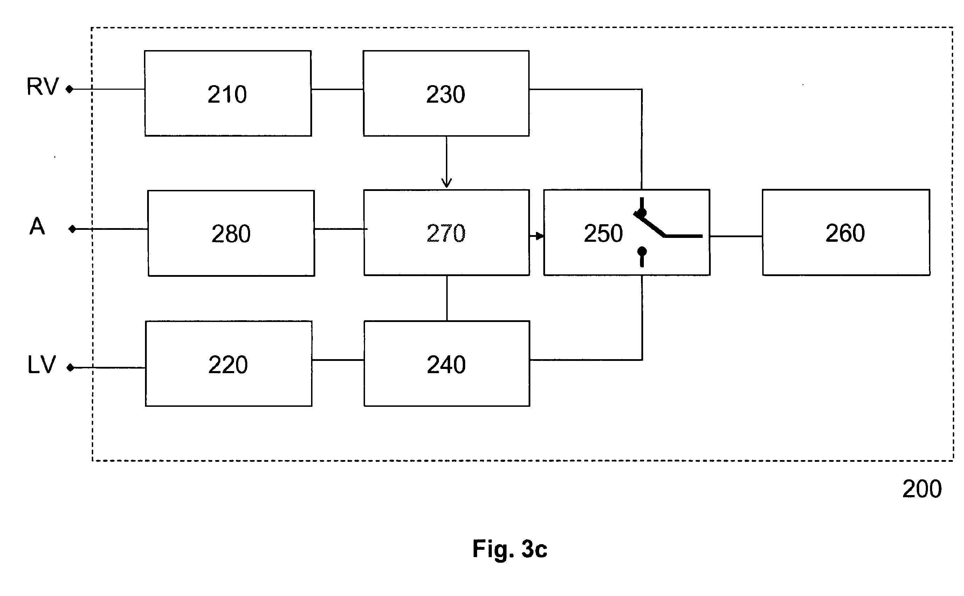

Außerdem kann die Bewertungseinheit nicht nur mit dem linksventrikulären EKG-Signalverstärker 220, sondern auch mit dem rechtsventrikulären EKG-Signalverstärker 210 und einem rechtsatrialen EKG-Signalverstärker 280 verbunden sein, wie dies schematisch in Figur 3c angedeutet ist. Damit ergibt sich die Möglichkeit, weitere Kriterien für das Umschalten zwischen primär rechtsventrikulärer Steuerung und primär linksventrikulärer Steuerung zu berücksichtigen, wie dies weiter unten noch näher dargestellt ist.

Das in den Figuren 3a bis c gezeigte Blockschaltbild kann zusätzlich um einen oder zwei atriale Sensingkanäle erweitert werden. Ebenso kann bei der Ausgestaltung des Herzstimulators als ICD ein zusätzlicher Arrhythmieklassifikator vorgesehen sein, der ebenfalls über einen Umschalter wahlweise an die RV- oder LV-Sensingstufe geschaltet wird. In diesem Falle ist die Umschaltung vorzugsweise unabhängig von der des Schrittmacherzeitgebers, so dass ein zweiter Umschalter vor dem Arrhythmieklassifikator zusätzlich vorgesehen sein kann.

Bevorzugt ist die Umschaltung jedoch in den Schrittmacherzeitgeber 260 integriert, so dass zunächst die Signale sowohl der rechtsventrikulären Elektrodenleitung RV als auch der linksventrikulären Elektrodenleitung LV dem Schrittmacherzeitgeber 260 verfügbar sind, die Umschaltung jedoch die Signalverarbeitung im Schrittmacherzeitgeber 260 beeinflusst. So ist es möglich, neben den Ereignissen des primären Steuerkanals auch die Ereignisse des jeweils anderen Kanals, z.B. zur Inhibierung von Stimulationsimpulsen, zu berücksichtigen.

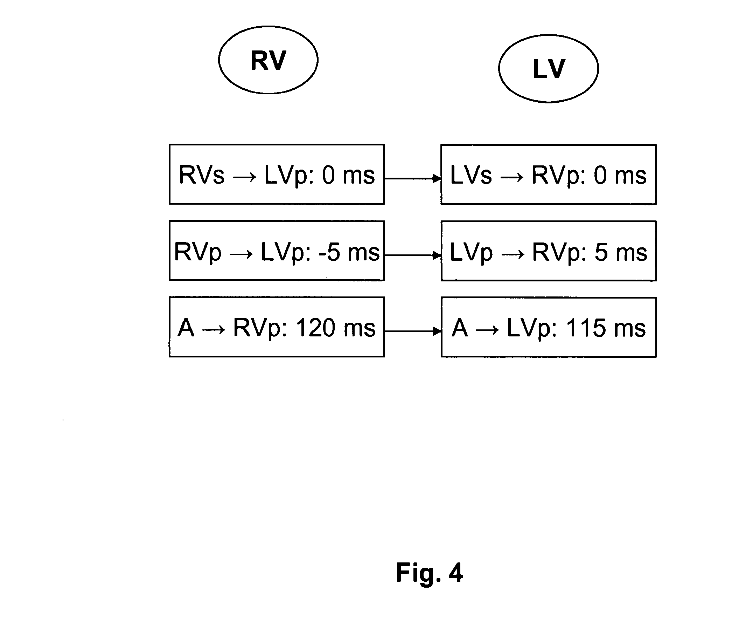

In Figur 4 ist ein Beispiel einer Parametertransformation bei Umschaltung von einem primär rechtsventrikulär gesteuerten Zeitgeber zu einem linksventrikulär gesteuerten Zeitgeber dargestellt. Diese Parametertransformation stellt sicher, dass die zeitlichen Beziehungen der biventrikulären Stimulation bei der Umschaltung erhalten bleiben. Idealerweise wird diese Parametertransformation in einem Regelwerk im Schrittmacherzeitgeber oder einem Programmiergerät hinterlegt, so dass bei der Umschaltung diese Parameteranpassung automatisch angewendet werden kann.

Bei der primär RV-gesteuerten Stimulation wird der LV-Stimulus (LVp) in Abhängigkeit des RV-Sense (RVs) oder des RV-Stimulus (RVp) mit einer entsprechenden VV-Zeit ausgelöst. Die RV-Stimulation (RVp) wird durch ein atriales Ereignis (A) gestartet, verzögert um eine Überleitungszeit von 120ms.

Wird der biventrikuläre Stimulator nun auf einen primär linksventrikulär gesteuerten Schrittmacherzeitgeber umgeschaltet, so werden die im Beispiel genannten Zeitgeberparameter automatisch im Schrittmacherzeitgeber transformiert. Die rechtsventrikuläre Stimulation (RVp) folgt nun dem linksventrikulären Ereignis (LVs oder LVp), wobei die zeitliche Beziehung so transformiert wird, dass die eigentlichen Stimulationszeitpunkte beim Übergang von einer primär rechtsventrikulär gesteuerten zu einer primär linksventrikulär gesteuerten Steuerung unverändert bleiben. Gleiches gilt auch für die Auslösung der linksventrikulären Stimulation (LVp). Hier wird im Beispiel die AV-Überleitungszeit um das VV-Delay (5ms) entsprechend korrigiert.

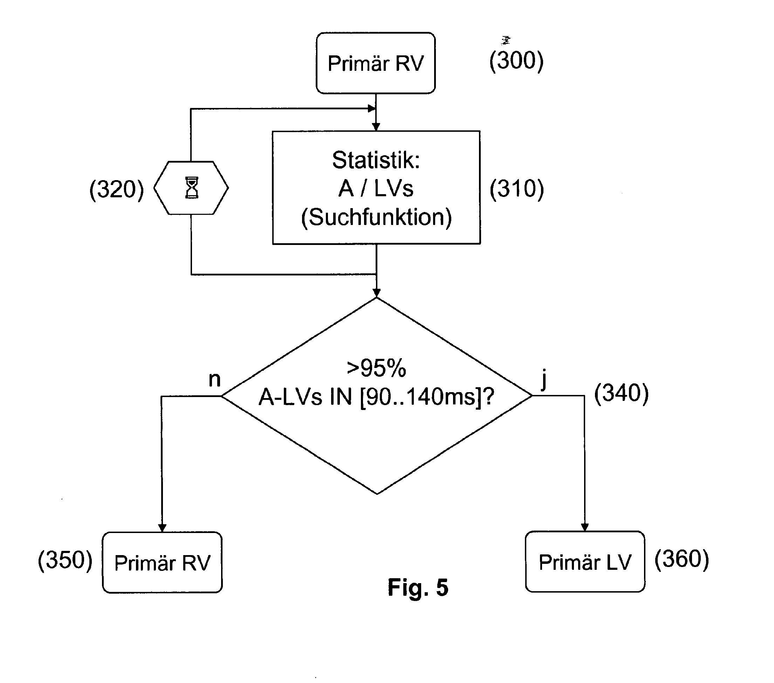

In Figur 5 ist der Ablauf einer automatischen Umschaltung von primär RV- nach primär LV-gesteuerter Stimulation nach Implantation dargestellt.

Unmittelbar nach der Implantation der linksventrikulären Elektrodenleitung LV ist der Herzstimulator immer primär rechtsventrikulär (300) gesteuert.

Um die Stabilität der linksventrikulären Elektrodenleitung LV zu beurteilen, wird eine Statistik der linksventrikulären Ereignisse, bezogen auf die atrialen Ereignisse, aufgezeichnet (310). Die kann z.B. durch ein Histogramm der A-LVs-Zeiten realisiert sein und durch eine Suchfunktion für intrinsische linksventrikuläre Ereignisse ergänzt werden.

Diese Aufzeichnung wird für eine programmierbare Zeit (320), z.B. für 12 Wochen, durchgeführt, um so ein Einwachsen der linksventrikulären Elektrodenleitung LV abzuwarten.

Ist diese Zeit (320) abgelaufen, wird geprüft, ob die aufgezeichnete A-LVs-Statistik eine stabile linksventrikuläre Elektrodenposition bestätigt. Dies kann z.B. dadurch erfolgen, dass geprüft wird, ob mehr als 95% der aufgezeichneten Ereignisse in einer definierten Zeitspanne liegen (340). Ist dies der Fall, dann wird automatisch auf die primäre linksventrikuläre Stimulation (360) umgeschaltet. Ist die Bedingung nicht erfüllt, dann bleibt das System bei der primär rechtsventrikulären Steuerung (350).

In einer erweiterten Ausführung werden zur Beurteilung der linksventrikulären Elektrodenstabilität die wahrgenommenen linksventrikulären Signalamplituden in einem typischen Amplitudenbereich für linksventrikulären Signale (z.B. >8mV) geprüft. Hier müssen z.B. X aus Y gemessenen linksventrikulären Amplituden einen Maximalwert größer 8mV erreichen und der Minimalwert darf nicht kleiner als 3mV sein. Zusätzlich kann dieses Amplitudenkriterium mit einem Vergleich der Amplitude des im rechten Ventrikels aufgenommenen Signals (rechtsventrikuläre Amplitude) mit der Amplitude des im linken Ventrikels aufgenommen Signals (linksventrikuläre Amplitude) ergänzt werden. Die Umschaltung auf eine primär linksventrikuläre Steuerung erfolgt nur dann, wenn die linksventrikuläre Amplitude größer als die rechtsventrikuläre Amplitude ist.

Eine weitere Verbesserung der linksventrikulären Elektrodenstabilitätsbewertung kann durch die Auswertung der morphologischen Signalcharakteristika des linksventrikulären Signals erreicht werden. Bevorzugt wird für die morphologische Auswertung ein ungefiltertes Signal, abgeleitet in einem parallelen Wahrnehmungskanal, bewertet. Bei bestehendem Eigenrhythmus (bzw. mit Suchfunktion nach intrinsischen Ereignissen) können hierfür die Parameter QRS-Breite im linksventrikulären Signal, Slew-Rate im linksventrikulären Signal, Integral des QRS-Komplexes, oder aber die Frequenzinhalte des linksventrikulären Signals, ausgewertet werden. In allen diesen Parametern unterscheidet sich ein linksventrikuläres Signal von einem atrialen Signal, so dass eine gute Differenzierung erreicht werden kann und eine in den Bereich des Vorhofs dislozierte linksventrikuläre Elektrodenleitung gut erkannt wird.

Für die Morphologiebewertung bei Stimulation kommt das von der LV-Capture-Bewertung bekannte Verfahren zum Einsatz. Hier kann sehr gut differenziert werden, ob die LV-Elektrode stabil implantiert oder disloziert ist. Zusätzlich kann die gemessene LV-Reizschwelle als Kriterium ergänzt werden. D.h. die Lage der linksventrikulären Elektrodenleitung wird nur dann als stabil betrachtet, wenn die Reizschwelle in einem vordefinierten Erwartungsfenster liegt.

Um eine gute Sensitivität und Spezifität für die linksventrikuläre Elektrodenstabilitätserkennung zu erreichen, können alle genannten Verfahren (A-LV-Zeitkriterium, Amplitudenkriterium und Morphologiekriterium) wie folgt kombiniert werden:
- Umschaltung auf eine primär linksventrikuläre Steuerung erfolgt, wenn alle 3 Kriterien erfüllt sind;
- Rückschaltung auf eine primär rechtsventrikuläre Steuerung erfolgt, wenn 2 aus 3 Kriterien nicht mehr erfüllt sind.

## Patentansprüche

1. Biventrikulärer Herzstimulator (100) mit
- einer rechtsventrikulären Sensingeinheit (210, 230), die einen Anschluss für eine rechtsventrikuläre Wahrnehmungselektrode (110) aufweist oder mit einer solchen verbunden ist,
- einer linksventrikulären Sensingeinheit (220,240), die einen Anschluss für eine links-ventrikuläre Wahmehmungselektrode (120) aufweist oder mit einer solchen verbunden ist, und die ausgebildet ist, linksventrikuläre Ereignisse zu detektieren,
- einer rechtsatrialen Sensingeinheit, die einen Anschluss für eine rechtsatriale Wahmehmungselektrode (130) aufweist oder mit einer solchen verbunden ist, und die ausgebildet ist, rechtsatriale Ereignisse zu detektieren,
- einem Schrittmacherzeitgeber (260), der mit der rechtsventrikulären Sensingeinheit und der linksventrikulären Sensingeinheit verbunden ist,
- einen programmierbaren automatischem Umschalter (250), der mit dem Schrittmacherzeitgeber wirkverbunden und ausgebildet ist, den Schrittmacherzeitgeber wahlweise zwischen einer primär rechtsventrikulären Steuerung und einer primär linksventrikulären Steuerung umzuschalten, sowie
- eine Bewertungseinheit (270), die mit dem Umschalter wirkverbunden und ausgebildet ist, wenigstens einen für eine Stabilität der Elektrodenlage der linksventrikulären Wahrnehmungselektrode charakteristischen Stabilitätsparameter zu erfassen und auszuwerten, wobei der programmierbare automatische Umschalter ausgebildet ist, die Schrittmacherzeitgebersteuerung in Abhängigkeit eines Wertes des erfassten Stabilitätsparameters automatisch umzuschalten,
**dadurch gekennzeichnet,**
**dass** die Bewertungseinheit zusätzlich mit der rechtsatrialen und der linksventrikulären Sensingeinheit verbunden und ausgebildet ist, als weiteren Stabilitätsparameter eine zeitliche Beziehung zwischen einander zugeordneten rechtsatrialen Ereignissen und linksventrikulären Ereignissen auszuwerten.

2. Biventrikulärer Herzstimulator nach Anspruch 1, **dadurch gekennzeichnet, dass** der Umschalter Bestandteil des Schrittmacherzeitgebers ist und so angeordnet und ausgebildet ist, dass zunächst die Signale sowohl der rechtsventrikulären Elektrodenleitung (RV) als auch der linksventrikulären Elektrodenleitung (LV) dem Schrittmacherzeitgeber (260) verfügbar sind, die Umschaltung jedoch die Signalverarbeitung im Schrittmacherzeitgeber 260 beeinflusst.

3. Biventrikulärer Herzstimulator nach Anspruch 1 oder 2, **dadurch gekennzeichnet, dass** die linksventrikuläre Sensingeinheit einen linksventrikulären EKG-Signalverstärker (220) aufweist, der ein linksventrikuläres Eingangssignal für die Bewertungseinheit (270) liefert, und die Bewertungseinheit (270) mit dem linksventrikulären EKG-Signalverstärker (220) verbunden und ausgebildet ist, eine Maximalamplitude des linksventrikulären Eingangssignals als Stabilitätsparameter zu erfassen.

4. Biventrikulärer Herzstimulator nach Anspruch 3, **dadurch gekennzeichnet, dass** die Bewertungseinheit ausgebildet ist, den Wert der Maximalamplitude eines linksventrikulären Eingangssignals mit einem Schwellwert zu vergleichen und ein Umschalten des Umschalters zu bewirken, wenn das linksventrikuläre Eingangssignal den vorgegebenen Schwellwert wenigstens einmal überschreitet.

5. Biventrikulärer Herzstimulator nach Anspruch 4, **dadurch gekennzeichnet, dass** die Bewertungseinheit ausgebildet ist, ein Umschalten des Umschalters zu bewirken, wenn das linksventrikuläre Eingangssignal den vorgegebenen Schwellwert eine vorgegebene Anzahl X-Mal innerhalb einer vorgegebene Anzahl Y von Herzzyklen überschreitet.

6. Biventrikulärer Herzstimulator nach einem der Ansprüche 3 bis 5, **dadurch gekennzeichnet, dass** die rechtsventrikuläre Sensingeinheit einen rechtsventrikulären EKG-Signalverstärker (210) aufweist, der ein rechtsventrikuläres Eingangssignal für die Bewertungseinheit (270) liefert, und die Bewertungseinheit (270) zusätzlich mit dem rechtsventrikulären EKG-Signalverstärker (210) verbunden und ausgebildet ist, eine Maxmalamplitude des rechtsventrikulären Eingangssignals als weiteren Stabilitätsparameter zu erfassen.

7. Biventrikulärer Herzstimulator nach Anspruch 6, **dadurch gekennzeichnet, dass** die Bewertungseinheit ausgebildet ist, den Wert der Maximalamplitude eines jeweiligen linksventrikulären Eingangssignals mit dem Wert der Maximalamplitude eines jeweiligen rechtsventrikulären Eingangssignals zu vergleichen und ein Umschalten des Umschalters hin zu einer primär linksventrikulären Steuerung zu bewirken, wenn der Wert der Maximalamplitude des jeweiligen linksventrikulären Eingangssignals größer ist als der Wert der Maximalamplitude des jeweiligen rechtsventrikulären Eingangssignals.

8. Biventrikulärer Herzstimulator nach einem der Ansprüche 1 bis 7, **dadurch gekennzeichnet, dass** die Bewertungseinheit ausgebildet ist, wenigstens ein morphologisches Signalcharakteristikum als zusätzlichen Stabilitätsparameter auszuwerten.

9. Biventrikulärer Herzstimulator nach Anspruch 8, **dadurch gekennzeichnet, dass** die Bewertungseinheit ausgebildet ist, als morphologisches Signalcharakteristikum eines oder mehrere Signalcharakteriska aus der Gruppe: Breite eines QRS-Komplexes im linksventrikulären Eingangssignal, Slew-Rate des linksventrikulären Eingangssignals, Integral eines QRS-Komplexes im linksventrikulären Eingangssignal und Frequenzinhalte des linksventrikulären Eingangssignals zu erfassen und auszuwerten.

10. Biventrikulärer Herzstimulator nach Ansprüchen 3, und 8, **dadurch gekennzeichnet, dass** die Bewertungseinheit ausgebildet ist, ein Umschalten der Schrittmacherzeitgebersteuerung zu bewirken, wenn wenigstens zwei der folgenden Kriterien erfüllt sind:
- die Maximalamplitude des linksventrikulären Eingangssignal erfüllt wenigstens eine vorgegebene Bedingung,
- wenigstens ein morphologisches Signalcharakteristikum erfüllt wenigstens eine vorgegebene Bedingung und
- die zeitliche Beziehung zwischen einander zugeordneten rechtsatrialen Ereignissen und linksventrikulären Ereignissen erfüllt wenigstens eine vorgegebene Bedingung.

11. Biventrikulärer Herzstimulator nach einem der Ansprüche 1 bis 10, **dadurch gekennzeichnet, dass** der Schrittmacherzeitgeber dazu ausgebildet ist, eine Parametertransformation durchzuführen, wenn eine Umschaltung von primär rechtsventrikulärer Steuerung auf eine primär linksventrikuläre Steuerung erfolgt oder umgekehrt, wobei die Parametertransformation ausgestaltet ist, zeitliche Beziehungen der für eine biventrikuläre Stimulation relevanten Ereignisse zu erhalten.

## Claims

1. A biventricular cardiac stimulator (100) comprising
- a right-ventricular sensing unit (210, 230), which has or is connected to a terminal for a right-ventricular sensing electrode (110),
- a left-ventricular sensing unit (220, 240), which has or is connected to a terminal for a left-ventricular sensing electrode (120) and is designed to detect left-ventricular events,
- a right-atrial sensing unit, which has or is connected to a terminal for a right-atrial sensing electrode (130) and is designed to detect right-atrial events,
- a pacemaker timer (260), which is connected to the right-ventricular sensing unit and to the left-ventricular sensing unit,
- a programmable automatic switch (250), which is operatively connected to the pacemaker timer and is designed to switch the pacemaker timer selectively between a primarily right-ventricular control and a primarily left-ventricular control, and
- an evaluation unit (270), which is operatively connected to the switch and is designed to detect and evaluate at least one stability parameter that is characteristic of the stability of the electrode position of the left-ventricular sensing electrode, wherein the programmable automatic switch is designed to automatically switch the pacemaker timer control according to a value of the detected stability parameter,
**characterised in that**
the evaluation unit is additionally connected to the right-atrial sensing unit and to the left-ventricular sensing unit and is designed to evaluate, as a further stability parameter, a temporal relationship between correlated right-atrial events and left-ventricular events.

2. The biventricular cardiac stimulator according to Claim 1, **characterised in that** the switch is part of the pacemaker timer and is arranged and designed such that the signals both of the right-ventricular electrode line (RV) and of the left-ventricular electrode line (LV) are initially available to the pacemaker timer (260), but the switch influences the signal processing in the pacemaker timer (260).

3. The biventricular cardiac stimulator according to Claim 1 or 2, **characterised in that** the left-ventricular sensing unit has a left-ventricular ECG signal amplifier (220), which supplies a left-ventricular input signal for the evaluation unit (270), and the evaluation unit (270) is connected to the left-ventricular ECG signal amplifier (220) and is designed to detect a maximum amplitude of the left-ventricular input signal as a stability parameter.

4. The biventricular cardiac stimulator according to Claim 3, **characterised in that** the evaluation unit is designed to compare the value of the maximum amplitude of a left-ventricular input signal with a threshold value and to trigger switching of the switch when the left-ventricular input signal exceeds the predefined threshold value at least once.

5. The biventricular cardiac stimulator according to Claim 4, **characterised in that** the evaluation unit is designed to trigger switching of the switch when the left-ventricular input signal exceeds the predefined threshold value a predefined number (X) of times within a predefined number (Y) of cardiac cycles.

6. The biventricular cardiac stimulator according to one of Claims 3 to 5, **characterised in that** the right-ventricular sensing unit has a right-ventricular ECG signal amplifier (210), which supplies a right-ventricular input signal for the evaluation unit (270), and the evaluation unit (270) is additionally connected to the right-ventricular ECG signal amplifier (210) and is designed to detect the maximum amplitude of the right-ventricular input signal as a further stability parameter.

7. The biventricular cardiac stimulator according to Claim 6, **characterised in that** the evaluation unit is designed to compare the value of the maximum amplitude of a respective left-ventricular input signal with the value of the maximum amplitude of a respective right-ventricular input signal and to trigger switching of the switch to a primarily left-ventricular control when the value of the maximum amplitude of the respective left-ventricular input signal is greater than the value of the maximum amplitude of the respective right-ventricular input signal.

8. The biventricular cardiac stimulator according to one of Claims 1 to 7, **characterised in that** the evaluation unit is designed to evaluate at least one morphological signal characteristic as an additional stability parameter.

9. The biventricular cardiac stimulator according to Claim 8, **characterised in that** the evaluation unit is designed to detect and evaluate, as a morphological signal characteristic, one or more signal characteristics from the group: width of a QRS complex in the left-ventricular input signal, slew rate of the left-ventricular input signal, integral of a QRS complex in the left-ventricular input signal, and frequency contents of the left-ventricular input signal.

10. The biventricular cardiac stimulator according to Claims 3 and 8, **characterised in that** the evaluation unit is designed to trigger switching of the pacemaker timer control when at least two of the following criteria are met:
- the maximum amplitude of the left-ventricular input signal satisfies at least one predefined condition,
- at least one morphological signal characteristic satisfies at least one predefined condition, and
- the temporal relationship between correlated right-atrial events and left-ventricular events satisfies at least one predefined condition.

11. The biventricular cardiac stimulator according to one of Claims 1 to 10, **characterised in that** the pacemaker timer is designed to perform a parameter transformation when switching from primarily right-ventricular control to primarily left-ventricular control or vice versa, wherein the parameter transformation is designed to maintain temporal relationships of the events relevant for biventricular stimulation.

## Revendications

1. Stimulateur cardiaque biventriculaire (100) comprenant
- une unité de détection de ventricule droit (210, 230), qui a ou est connectée à une borne pour une électrode de détection de ventricule droit (110),
- une unité de détection de ventricule gauche (220, 240), qui a ou est connectée à une borne pour une électrode de détection de ventricule gauche (120) et est conçue pour détecter des événements de ventricule gauche,
- une unité de détection d'oreillette droite, qui a ou est connectée à une borne pour une électrode de détection d'oreillette droite (130) et est conçue pour détecter des événements d'oreillette droite,
- un temporisateur de pacemaker (260), qui est connecté à l'unité de détection de ventricule droit et à l'unité de détection de ventricule gauche,
- un commutateur automatique programmable (250), qui est connecté de façon fonctionnelle au temporisateur de pacemaker et est conçu pour commuter le temporisateur de pacemaker de façon sélective entre une commande de ventricule droit principalement et une commande de ventricule gauche principalement ; et
- une unité d'évaluation (270), qui est connectée de façon fonctionnelle au commutateur et est conçue pour détecter et évaluer au moins un paramètre de stabilité qui est caractéristique de la stabilité de la position d'électrode de l'électrode de détection de ventricule gauche, le commutateur automatique programmable étant conçu pour commuter automatiquement la commande de temporisateur de pacemaker conformément à une valeur du paramètre de stabilité détecté,
**caractérisé par le fait que**
l'unité d'évaluation est additionnellement connectée à l'unité de détection d'oreillette droite et à l'unité de détection de ventricule gauche et est conçue pour évaluer, comme autre paramètre de stabilité, une relation temporelle entre des événements d'oreillette droite et des événements de ventricule gauche corrélés.

2. Stimulateur cardiaque biventriculaire selon la Revendication 1, **caractérisé par le fait que** le commutateur fait partie du temporisateur de pacemaker et est disposé et conçu de telle sorte que les signaux à la fois de la ligne d'électrode de ventricule droit (RV) et de la ligne d'électrode de ventricule gauche (LV) sont initialement disponibles pour le temporisateur de pacemaker (260), mais le commutateur influence le traitement de signal dans le temporisateur de pacemaker (260).

3. Stimulateur cardiaque biventriculaire selon l'une des Revendications 1 ou 2, **caractérisé par le fait que** l'unité de détection de ventricule gauche a un amplificateur de signaux d'ECG de ventricule gauche (220), qui fournit un signal d'entrée de ventricule gauche pour l'unité d'évaluation (270), et que l'unité d'évaluation (270) est connectée à l'amplificateur de signaux d'ECG de ventricule gauche (220) et est conçue pour détecter une amplitude maximale du signal d'entrée de ventricule gauche en tant que paramètre de stabilité.

4. Stimulateur cardiaque biventriculaire selon la Revendication 3, **caractérisé par le fait que** l'unité d'évaluation est conçue pour comparer la valeur de l'amplitude maximale d'un signal d'entrée de ventricule gauche à une valeur de seuil et pour déclencher une commutation du commutateur lorsque le signal d'entrée de ventricule gauche dépasse la valeur de seuil prédéfinie au moins une fois.

5. Stimulateur cardiaque biventriculaire selon la Revendication 4, **caractérisé par le fait que** l'unité d'évaluation est conçue pour déclencher une commutation du commutateur lorsque le signal d'entrée de ventricule gauche dépasse la valeur de seuil prédéfinie un nombre prédéfini (X) de fois à l'intérieur d'un nombre prédéfini (Y) de cycles cardiaques.

6. Stimulateur cardiaque biventriculaire selon l'une des Revendications 3 à 5, **caractérisé par le fait que** l'unité de détection de ventricule droit a un amplificateur de signaux d'ECG de ventricule droit (210), qui fournit un signal d'entrée de ventricule droit pour l'unité d'évaluation (270), et que l'unité d'évaluation (270) est en outre connectée à l'amplificateur de signaux d'ECG de ventricule droit (210) et est conçue pour détecter l'amplitude maximale du signal d'entrée de ventricule droit en tant qu'autre paramètre de stabilité.

7. Stimulateur cardiaque biventriculaire selon la Revendication 6, **caractérisé par le fait que** l'unité d'évaluation est conçue pour comparer la valeur de l'amplitude maximale d'un signal d'entrée de ventricule gauche respectif à la valeur de l'amplitude maximale d'un signal d'entrée de ventricule droit respectif et pour déclencher une commutation du commutateur à une commande de ventricule gauche principalement lorsque la valeur de l'amplitude maximale du signal d'entrée de ventricule gauche respectif est supérieure à la valeur de l'amplitude maximale du signal d'entrée de ventricule droit respectif.

8. Stimulateur cardiaque biventriculaire selon l'une des Revendications 1 à 7, **caractérisé par le fait que** l'unité d'évaluation est conçue pour évaluer au moins une caractéristique de signal morphologique en tant que paramètre de stabilité supplémentaire.

9. Stimulateur cardiaque biventriculaire selon la Revendication 8, **caractérisé par le fait que** l'unité d'évaluation est conçue pour détecter et évaluer, en tant que caractéristique de signal morphologique, une ou plusieurs caractéristiques de signal du groupe : largeur d'un complexe QRS dans le signal d'entrée de ventricule gauche, vitesse de balayage du signal d'entrée de ventricule gauche, intégrale d'un complexe QRS dans le signal d'entrée de ventricule gauche, et contenus fréquentiels du signal d'entrée de ventricule gauche.

10. Stimulateur cardiaque biventriculaire selon l'une des Revendications 3 et 8, **caractérisé par le fait que** l'unité d'évaluation est conçue pour déclencher une commutation de la commande de temporisateur de pacemaker lorsqu'au moins deux des critères suivants sont satisfaits :
- l'amplitude maximale du signal d'entrée de ventricule gauche satisfait au moins une condition prédéfinie,
- au moins une caractéristique de signal morphologique satisfait au moins une condition prédéfinie ; et
- la relation temporelle entre les événements d'oreillette droite et les événements de ventricule gauche corrélés satisfait au moins une condition prédéfinie.

11. Stimulateur cardiaque biventriculaire selon l'une des Revendications 1 à 10, **caractérisé par le fait que** le temporisateur de pacemaker est conçu pour effectuer une transformation de paramètre lors de la commutation de la commande de ventricule droit principalement à la commande de ventricule gauche principalement ou réciproquement, la transformation du paramètre étant conçue pour maintenir des relations temporelles des événements pertinents pour la stimulation biventriculaire.
